# EUROPEAN PATENT APPLICATION

(11) **EP 4 710 863 A1**
(43) Date of publication of application: **18.03.2026**
(21) Application number: 24199906.9
(22) Date of filing: 12.09.2024
(51) Int. Cl.: A61B 8/08, A61B 8/00, G06T 7/00, G16H 50/20

(54) **PLACENTAL IMAGING**

(71) Applicant: Koninklijke Philips N.V., 5656 AG Eindhoven (NL)
(72) Inventor: ANURADHA, Anuradha, Eindhoven (NL); KULKARNI, Chaitanya, Eindhoven (NL); SETH, Subhendu, 5656AG Eindhoven (NL); GUPTA, Madhumita, Eindhoven (NL); V, Manikanda Krishnan, Eindhoven (NL); N, Narendra, Eindhoven (NL)
(74) Representative: Philips Intellectual Property & Standards

(57) **Abstract**

A placental imaging system comprises a hand held ultrasound imager for scanning over the lower abdomen of a patient and configured to generate image data. An AI algorithm is used to identify the location and shape of the placenta and cervix within the image data and determine the placenta to internal OS distance. It is thereby determined if the placenta has a positional abnormality, such as if it is low lying or not. A recommendation system receives a gestational age of the patient and the positional abnormality information to recommend a patient action.

## Description

### FIELD OF THE INVENTION

This invention relates to the imaging of the placenta to detect placental abnormalities.

### BACKGROUND OF THE INVENTION

The placenta is crucial for pregnancy. It has indispensable functions in the development and protection of the fetus.

Placental abnormalities with respect to location and anatomy in pregnancy include low-lying placentas, placenta previa and abnormally invasive placentas. These conditions form a risk of antepartum, intrapartum, and postpartum hemorrhage.

Assessment of placental position and its relation to the internal cervical OS (herein "internal OS") is one of the important aspects of obstetric ultrasound imaging. The location of the placenta relative to the internal OS (previa, low-lying, or distant from the cervical canal), may be determined as early as the beginning of the second trimester, usually during the early anatomic scan.

A low-lying placenta (LLP) is diagnosed when the inferior placental edge is within 2 cm of the internal OS but does not overlie it. Previa is diagnosed when the inferior placental edge covers the internal OS, either partially or completely, sometimes crossing over to the other side of the OS. Resolution of low placentation, when diagnosed in the mid-trimester, occurs in 66% to 98% of cases and is more likely if it is low-lying rather than placenta previa.

There is therefore a need to identify positional abnormality of the placenta, such as a low-lying placenta. There are increased chances of delivery by C-section when there is detection of low lying placenta.

The current method of identifying low lying placenta and placenta previa is using a comprehensive scan with a trained ultrasound sonographer. Ultrasound imaging is a widely used diagnostic tool for obstetric imaging which provides valuable insights into anatomical structures but may require manual interpretation and measurement by skilled professionals.

The assessment of the low lying placenta and previa needs expertise to identify the distance from the cervix (internal OS) to the lower edge of the placenta. The availability of this expertise may not always be feasible in low resource settings and in a busy community hospital with overloaded patient population.

There is therefore a need for an improved way in particular to identify low lying placenta which can reduce the amount of expertise needed.

### SUMMARY OF THE INVENTION

The invention is defined by the claims.

According to examples in accordance with an aspect of the invention, there is provided a placental imaging system comprising:
a hand held ultrasound imager for scanning over the lower abdomen of a patient and configured to generate image data;
an image analysis system comprising an AI algorithm configured to:
   use image segmentation to identify the location and shape of the placenta and cervix within the image data;
   use the cervix location and shape to identify a position of the internal OS; and
   use the placenta location and shape to determine the placenta to internal OS distance and to generate a placental positional abnormality indication; and
a recommendation system for receiving a gestational age of the patient and the placental positional abnormality indication and configured to recommend a patient action.

This imaging system uses an AI algorithm (e.g. deep learning algorithm) to identify the location and shape of the placenta and cervix. A segmentation algorithm may be used for this purpose. The cervix segmentation is used to determine the internal OS and the proximity to the placenta may then be derived from a placenta segmentation. The recommendation system performs patient categorization, i.e. patient prioritization, based on the gestational age and risk of a positional abnormality such as a low-lying placenta. Patients may for example be categorized for patient action comprising urgent investigation or else routine follow-up.

The placental positional abnormality indication for example indicates if the placenta is low lying or not. However, other positional abnormalities may also be detected.

For example, the recommendation system may be configured to recommend a patient action comprising:
immediate follow up if the gestational age is above a threshold and the placenta is indicated as low lying;
priority follow up if the gestational age is below the threshold and the placenta is indicated as low lying; and
normal follow up timing if the placenta is indicated as not low lying.

Advancements in artificial intelligence (AI) and image processing have opened new possibilities for automating and enhancing diagnostic workflows. The AI-based solution of this disclosure identifies such high-risk patients with low lying placenta, and it thereby enables less skilled healthcare workers like nurse-midwives or junior doctors not trained in ultrasound, or resident doctors in training, to identify this condition and immediately refer these mothers for management at later gestational ages or schedule a follow up scan appropriately to assess the migration of the placenta.

The method for example uses AI-guided detection of the cervix and lower edge of the placenta and their positional relationship to each other, to predict the possibility of a low lying placenta. This may be achieved by means of a single horizontal sweep of a hand held ultrasound probe.

Patients who are at no risk of placenta previa or low lying placenta can be filtered out, to reduce hospital burden. This solution also enables patients to be prioritized for detailed obstetric ultrasound scans based on how close the placenta is detected to the internal OS of the cervix.

The prioritization is performed based on a handheld ultrasound probe, and hence the solution is suitable for low resource settings. This solution can be used not just by a sonographer, but any medical professional trained on the imaging system.

The hand held ultrasound imager is for example further configured to generate sensor data that indicates the orientation of the hand held ultrasound imager; The sensor data may further indicate the speed of movement of the hand held ultrasound imager.

This sensing enables a guidance function to be performed, in that the scan procedure followed by the user can be analyzed. Thus, the imaging system may further comprise a guidance system for guiding a user to scan the hand held ultrasound imager over the lower abdomen of the patient based on the sensor data.

The guidance system is for example configured to perform an image quality check by comparing the sensor data with a scan protocol.

The guidance system for example implements a guided sweep solution based on a YOLO (you only look once) object detection approach, to help the user to collect the desired image accurately. The image quality check is for example also based on YOLO.

The AI algorithm for example comprises a real-time anatomical feature detection model that is configured to identify the placenta and cervix. The real-time anatomical feature detection model may be further configured to identify the maternal urinary bladder. The anatomical feature detection model for example comprises a single shot detection algorithm.

The AI algorithm may also comprise a segmentation model that is configured to segment the placenta, cervix and uterus. The AI algorithm is for example configured to identify a position of the internal OS as a point on the periphery of the cervix segmentation.

In one example, the segmentation model is configured to identify the internal OS and determine the placenta to internal OS distance by:
defining a bounding box around the cervix segmentation;
forming a diagonal line through the bounding box;
identifying where the diagonal line meets the cervix segmentation boundary in a central imaging slice of the cervix, and defining this point as the internal OS;
defining a sphere around the internal OS;
determining if the placenta is within the sphere using the placenta segmentation.

In this way, after identifying the internal OS, it is determined if the segmentation of the placenta includes a part which lies within a fixed distance (e.g., 2 cm) of the internal OS.

This disclosure also provides a placental imaging method comprising:
receiving image data from a hand held ultrasound imager during scanning over the lower abdomen of a patient;
using an AI algorithm to:
   perform segmentation to identify the location and shape of the placenta and cervix within the image data;
   use the cervix location and shape to identify a position of the internal OS;
   use the placenta location and shape to determine the placenta to internal OS distance; and
   generate a placental positional abnormality indication;
receiving a gestational age of the patient and the placental positional abnormality indication; and
recommending a patient action based on the gestational age and the placental positional abnormality indication.

The method may further comprise receiving sensor data that indicates the orientation and speed of movement of the hand held ultrasound imager and the method comprises performing an image quality check by comparing the sensor data with a scan protocol.

Using the AI algorithm for example comprises:
using a real-time anatomical feature detection model to identify the placenta and cervix;
using a segmentation model to segment the placenta, cervix and uterus and identify the internal OS.

This disclosure also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a low lying placenta which is about 2-3 mm away from the internal OS of the cervix;
Fig. 2 shows how to measure the OS-lower placental edge distance;
Fig. 3 shows a placenta previa covering the internal OS of the cervix.
Fig. 4 shows a method of acquisition of the cervical sweep;
Fig. 5 indicates the approximate location of the scan on the abdomen.
Fig. 6 shows a flow diagram of the method implemented by the guidance system;
Fig. 7 shows the internal OS detection method, using the cervix segmentation;
Fig. 8 shows an example output of the flow for identifying the internal OS; and
Fig. 9 shows a placental imaging system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a placental imaging system comprising a hand held ultrasound imager for scanning over the lower abdomen of a patient and configured to generate image data. An AI algorithm is used to identify the location and shape of the placenta and cervix within the image data and determine the placenta to internal OS distance. It is thereby determined if the placenta has a positional abnormality, such as if it is low lying or not. A recommendation system receives a gestational age of the patient and the positional abnormality information to recommend a patient action.

In this way, the process of identifying a positional abnormality, such as a low lying placenta and previa in ultrasound images is automated and streamlined. This automation reduces the reliance on manual interpretation, the less skilled sonographers and health care professionals to detect this high-risk condition. This saves time for experts and improves workflow efficiency.

Improved patient care outcomes are provided by enabling early detection, monitoring, and optimizing fetal delivery strategies.

AI-based algorithms can analyze ultrasound images with high accuracy and precision, reducing interobserver variations. The ability to measure distance accurately aids in clinical decision making and treatment planning.

The AI algorithm can be integrated into ultrasound systems or imaging platforms to enhance their capabilities, offering advanced diagnostic tools to healthcare providers.

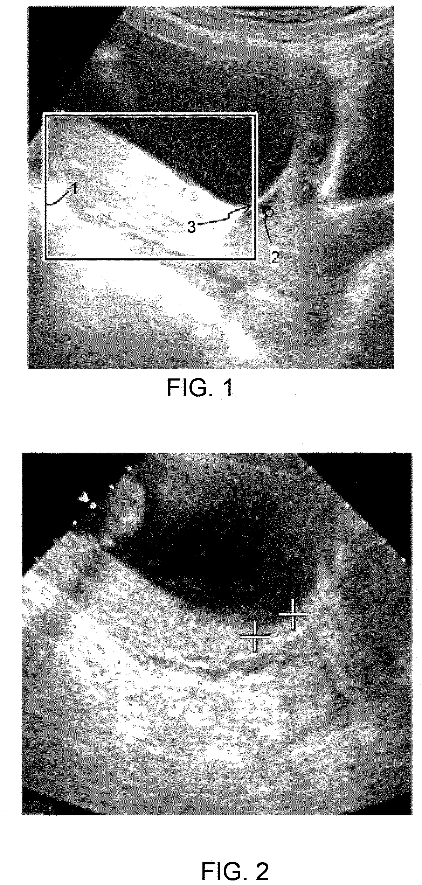

Fig. 1 shows a low lying placenta which is about 2-3 mm away from the internal OS of the cervix. The placenta bounding box is shown as rectangle 1, and the internal OS is shown as location 2. The lower edge of the placenta is shown as location 3.

Fig. 2 shows how to measure the internal OS to lower placental edge distance, by manually allocating cross hairs to the locations between which distance is to be measured.

This requires a skilled and highly trained sonographer.

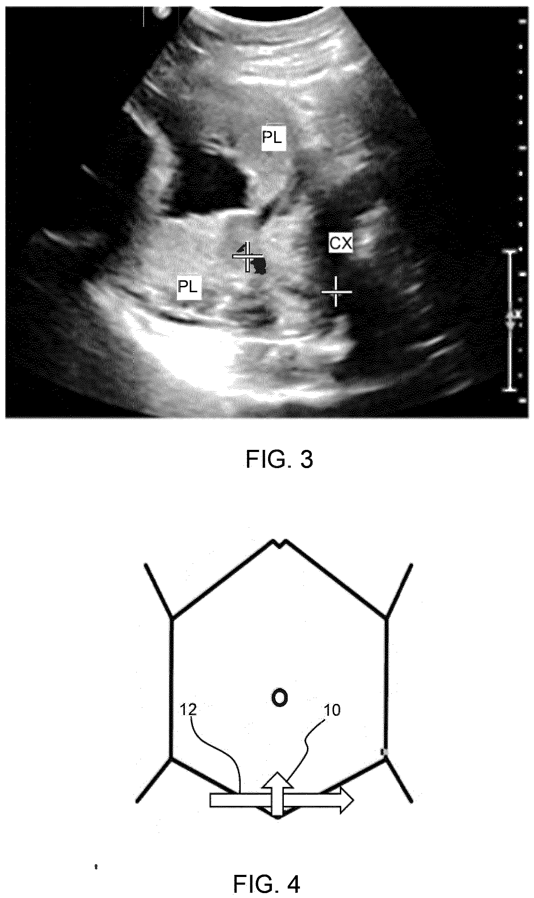

Fig. 3 shows a placenta previa in which the placenta (PL) covers the internal OS of the cervix (CX).

Fig. 4 shows a method of acquisition of the cervical sweep. The probe position and facing direction is represented by arrow 10, and the arrow 12 indicates the direction of the cervical sweep. The probe is placed at the lower most aspect of the abdomen in a rectangular area above the pubic symphysis. The cervical sweep is performed by traversing right to left of the patient.

The current method of identifying placenta previa uses a comprehensive scan with a trained sonographer.

The AI-based solution of this disclosure enables a guided sweep solution (for example based on a you only look once, YOLO, method, to help the user to collect the desired image accurately. An image quality check (again based on YOLO) using ultrasound sensors can then confirm if the scan protocol has been followed correctly or not.

An AI-based (deep learning or machine learning) segmentation algorithm is used to to accurately identify the location and shape of at least the placenta and cervix. Using the cervix segmentation algorithm, the internal OS can be determined. A circle (or sphere) may then be defined around the internal OS to identify a positional abnormality, such as if the placenta is low lying or not. A prioritization algorithm of a recommendation system, for example based on gestational age and risk of low lying placenta, can then categorize patients into urgent or routine follow-up.

In one implementation, the AI-algorithm comprises two deep learning algorithms which need data to be collected as per the defined protocol.

A scan is performed following the scan protocol on the lower abdominal region from right to left just above pubic symphysis. The lower uterine segment, placenta, and cervix have to be imaged in this scan.

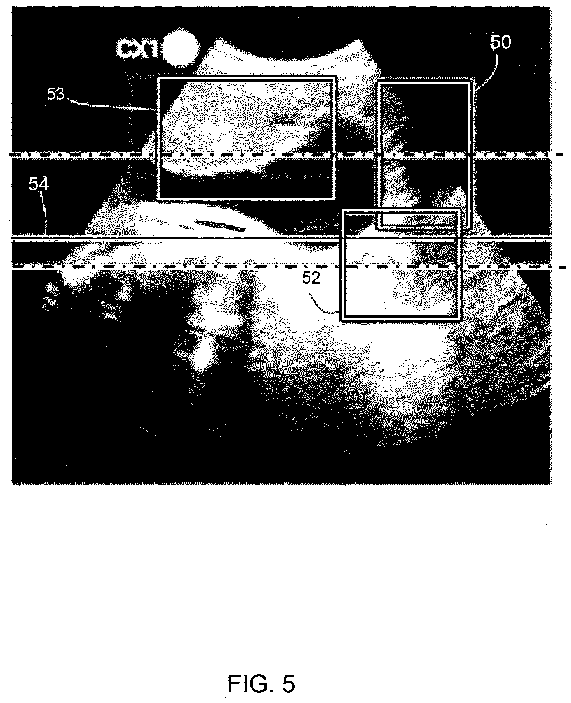

Fig. 5 indicates the approximate location of the scan on the abdomen.

The bounding box 50 shows the location of the maternal urinary bladder (MUB), and the bounding box 52 shows the location of the cervix. The bounding box 53 represents the detection of the placenta (by the YOLO algorithm). This data is collected in such way that the center of the MUB (shown as a dotted line through the center of box 50) is above the line 54 that indicates the center of the cone beam of the hand held ultrasound scanner and the center of the cervix (shown as a dottle line through the center of box 52) is below the line 54.

Along with the ultrasound image data (collected as a dicom file) ultrasound sensor data is also collected. This sensor data provides the angle at which the probe was kept on the abdominal surface and the speed at which the probe is moving. As shown in Fig. 5, the data collection should be such that the center of maternal urinary bladder is in the top half and the cervix is visible in the bottom half of the ultrasound cone beam.

Once the data collection is completed, the collected sensor data is used to identify the spacing between the slices of ultrasound images captured. The first slice captured will be marked as captured as position 0 and each slice captured will be marked as x cm from the first slice. This data collection should be done with the help of an expert ultrasound user to ensure proper sweep protocol is followed.

The AI-algorithm makes use of two trained models. The first is a detection model designed to run in real-time and the second is a segmentation model. This can take more time to infer.

For the development of the detection model, the annotation preferably defines bounding boxes of the following structures:
1. Placenta
2. Cervix
3. Maternal Urinary Bladder (MUB)

For the development of the segmentation model the annotation preferably identifies the following structures:
1. Cervix
2. Placenta
3. Uterus

Using the data collected, the first model, in particular a single shot detection (SSD) algorithm such as YOLO, is trained. This algorithm is optimized to run in near run-time to provide real-time guidance to the user. This SSD for example uses a combination of focal-loss and mean squared error (MSE) to optimize the classification and regression subnets.

Using the data collected, the second model is trained to accurately segment the cervix, placenta and uterus location. The trained model is any segmentation model (2D, 2.5D & 3D segmentation) that provides a high dice score. This model is for example trained with a weighted loss function of binary cross-entropy and dice score.

A sensor-based algorithm is used to gather the ultrasound sensor data after the scan. This data is processed to check if the data collection protocol was followed correctly or not. For example, acceleration data sensor is used to identify if the probe has been moved in the correct direction or not. Gyro sensor is used to make sure the angle has not been changed during the data acquisition.

If the sensor data is within the defined error limits, then the data is passed for further analysis. If the data collected has breached the error limits, a re-scan of the patient is requested. In this way, a guidance system is implemented for guiding a user to scan the hand held ultrasound imager over the lower abdomen of the patient based on the sensor data.

The AI algorithm is preferably used to implement a data quality check and thereby enable guidance to be provided to the user. For example, analysis of the sensor data from the ultrasound probe can determine if the probe wobbled or if was there back and forth movement with the probe when the data was collected. In this way, it can be verified if the data collected correctly matches the scan protocol.

This analysis is run in real-time and provides insight into where the placenta, MUB and cervix are located. If the MUB and cervix are not visible in the scanned images, a request for a re-scan is generated.

Along with requesting a re-scan, the analysis can also can provide inputs or guidance based on the following criteria (based on location of the cervix and MUB):
(i) If the MUB is not seen, but the cervix is seen, the guidance will be to re-scan slightly towards the top.
(ii) If the cervix is not seen, but the MUB is seen, the guidance will be to re-scan slightly towards the bottom. In both cases, the guidance will recommend that the MUB should ideally be seen towards the top of the image and the cervix should be seen towards the bottom of the image.

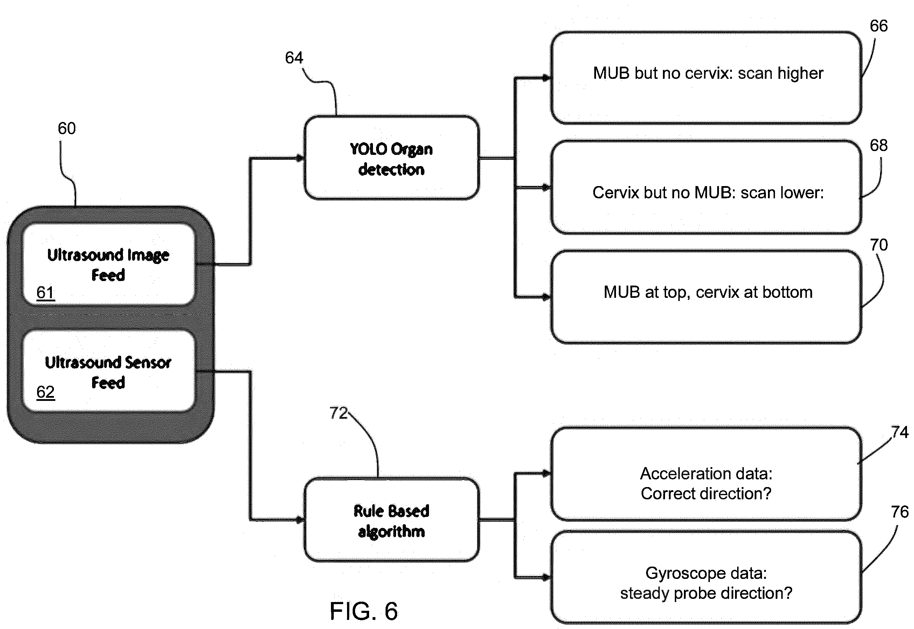

Fig. 6 shows a flow diagram of the method implemented by the guidance system, for guiding a user to scan the hand held ultrasound imager over the lower abdomen of the patient.

The hand held ultrasound imager 60 generates an ultrasound image feed 61 and an ultrasound sensor feed 62.

The image feed is provided to the detection algorithm 64, such as a YOLO detection algorithm for organ detection.

In step 66 it is determined if the MUB is not seen but the cervix is seen, in which case the guidance is to rescan slightly towards the top.

In step 68 it is determined if the cervix is not seen but the MUB is seen, in which case the guidance is to rescan slightly towards the bottom.

In step 70 guidance is provided to assist in locating the MUB towards the top of the image and the cervix towards the bottom of the image.

The sensor data is provided to a rule based algorithm 72.

In step 74, acceleration data is used to identify if the probe has been moved in the correct direction.

In step 76, gyroscope data is used to determine if the probe angle changed during the data acquisition.

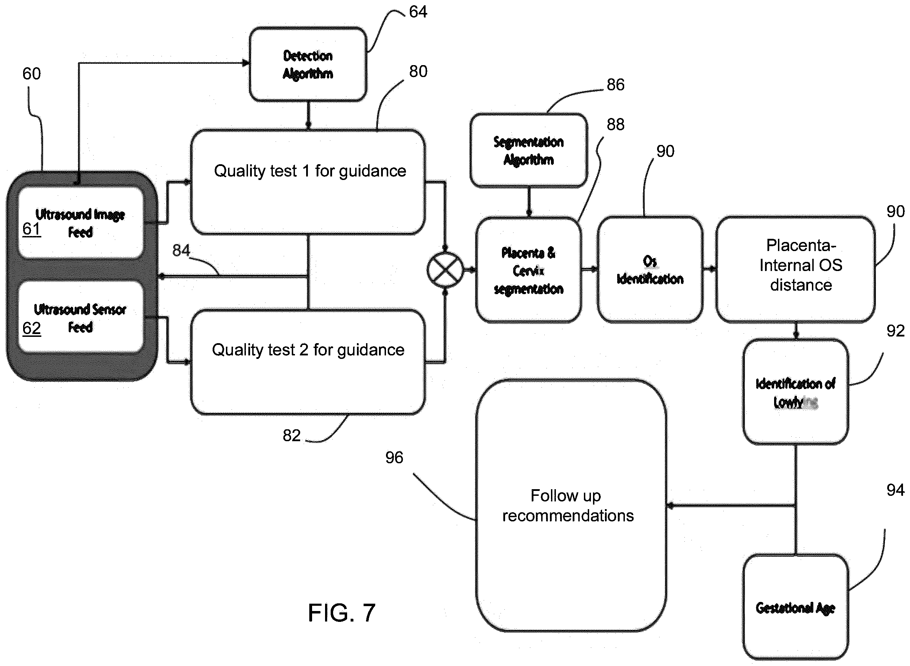

Fig. 7 shows the internal OS detection method, using the cervix segmentation.

The hand held ultrasound imager 60 is again shown, generating the ultrasound image feed 61 and the ultrasound sensor feed 62. The image feed is again provided to the detection algorithm 64, such as a YOLO detection algorithm for organ detection.

A first quality test 80 makes use of the image feed and a second quality test 82 makes use of the sensor data. These quality tests enable the guidance to be provided as explained above. If needed, a rescan trigger 84 is generated.

The segmentation algorithm 86 generates a placenta and cervix segmentation 88. The segmentation is used to provide identification of the internal OS in step 90.

One method of identifying the internal OS will be explained. For his method, first only the cervix segmentation is used from the raw segmentation output of the segmentation algorithm.

Once all the cervix segmentation regions have been detected, the largest connected component (cervix segmentation component) in the image is identified, the rest of the components are removed (because they are often false positives). Thus, this will help filter out false positives from the algorithm.

A boundary of the cervix segmentation is then obtained by first making a copy of the image, performing an erosion technique on the copied image and subtracting the eroded image from the actual image to obtain the boundary of the segmentation. The bounding box around the identified cervix is thereby derived.

A line is then fit from the top left corner of the bounding box to the bottom right corner. This defines a bounding box diagonal.

The central slice of the cervix is identified. This can be calculated from the ultrasound sensor data collected.

The central slice corresponds to one probe position along the left-right sweep of the ultrasound probe.

The point where the bounding box diagonal first touches the segmentation boundary on the central slice of the cervix is then marked as the position of internal OS for the cervix.

A sphere is then defined around from that point, and it is used to check if the placenta is within the given range. An additional margin of error can also be added to correct for error in the internal OS identification. This may be a tunable parameter.

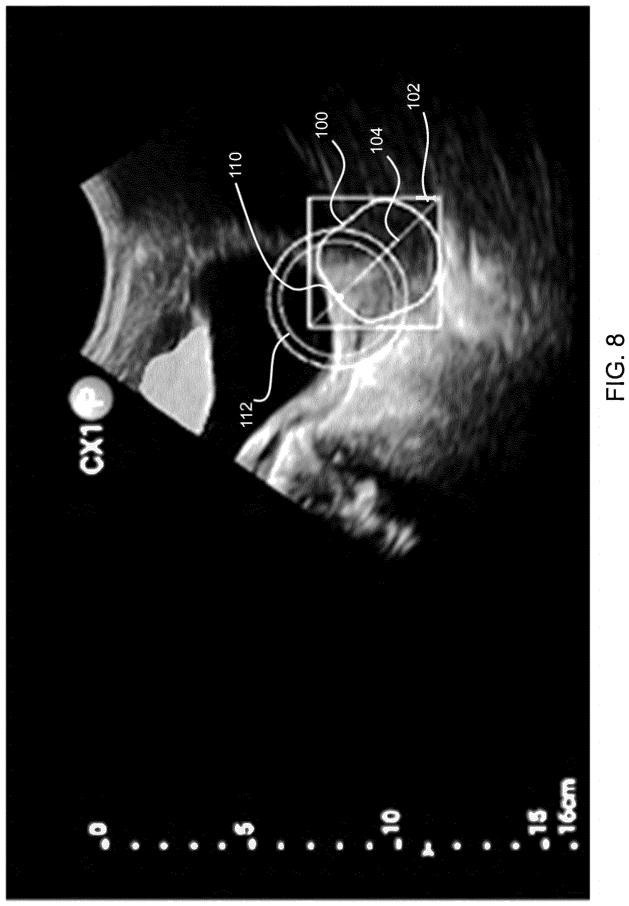

Fig. 8 shows an example output of the above-mentioned flow.

The cervix is shown as 100 with its bounding box 102 and diagonal 104. The touching point between the cervix segmentation 100 and the diagonal is shown as point 110 and this is the center of the 2 cm sphere 112. A larger sphere is also shown to provide a margine of error.

There are alternative methods for identifying the internal OS. For example, the internal OS can be identified by fitting a polynomial through the uterus passing through cervix centroid. If the quality of captured ultrasound improves in the future, segmenting the internal OS directly can also be an option.

Returning to Fig. 7, after internal OS identification in step 90, the distance between the placenta and the internal OS is calculated in step 92. For this purpose, the segmentation of the placenta is added back to the mask (of Fig. 8) and it is checked if it falls within the circle identified around the internal OS. If it falls within the range, it is marked as low lying in step 94, or else it is marked as non-low lying. The patient is thus marked as low lying or not.

In this implementation of the algorithm, 2D circles are used to threshold the measurements and a check is made until the cervix size reduces to e.g., 75% of the maximum circle size (in a maximum slice). This avoids the need for information about the inter-slice distance. In another implementation, a 3D sphere may be defined from the identified internal OS.

If the patient is found to have a low lying placenta, the recommendation to be provided by a recommendation system will be a function of the gestational age (GA) which is input at step 96.

In step 98, if the patient is marked as having a low lying placenta and has a high GA then immediate follow up is recommended (e.g., >28 weeks).

If the patient is marked as having a low lying placenta and has a lower GA, then a priority follow up is recommended (namely a follow up before the recommended next regular follow up is assigned at 32 weeks).

If the patient is marked as not having a low lying placenta, the follow up schedule is not changed.

These rules can be adapted to local regulations.

The system is for example implemented as a handheld ultrasound device connected to a smart device such as a Tab, smart phone or computer. The solution is for example run on a smart device as part of an application which can visualize the direct feed from the ultrasound.

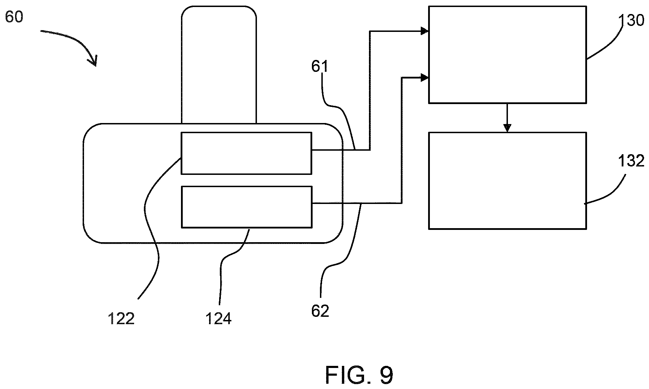

Fig. 9 shows a placental imaging system comprising a hand held ultrasound imager 60 for scanning over the lower abdomen. It comprises an ultrasound transducer 122 for generating image data 61 and sensors 124 for generating sensor data 62.

An image analysis system 130 comprising the AI algorithm explained above and a recommendation system 132 provides the categorization of the patient and provides recommendation for patient action based on the determination of whether the placenta is low lying or not (or more generally based on an indication of placental positional abnormality or normality).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner. (optional)

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A placental imaging system comprising:
a hand held ultrasound imager (60) for scanning over the lower abdomen of a patient and configured to generate image data (61);
an image analysis system (130) comprising an AI algorithm configured to:
use image segmentation to identify the location of the placenta and cervix within the image data;
use the cervix location to identify a position of the internal OS; and
use the placenta location to determine the placenta to internal OS distance and to generate a placental positional abnormality indication; and
a recommendation system (132) for receiving a gestational age of the patient and the placental positional abnormality indication and configured to recommend a patient action.

2. The imaging system of claim 1, wherein the placental positional abnormality indication indicates if the placenta is low lying or not.

3. The imaging system of claim 1 or 2, wherein the recommendation system is configured to recommend a patient action comprising:
immediate follow up if the gestational age is above a threshold and the placenta is indicated as low lying;
priority follow up if the gestational age is below the threshold and the placenta is indicated as low lying; and
normal follow up timing if the placenta is indicated as not low lying.

4. The imaging system of any one of claims 1 to 3, wherein the hand held ultrasound imager is further configured to generate sensor data (62) that indicates the orientation of the hand held ultrasound imager.

5. The imaging system of claim 4, wherein the sensor data (62) further indicates the speed of movement of the hand held ultrasound imager.

6. The imaging system of claim 4 or 5, further comprising a guidance system for guiding a user to scan the hand held ultrasound imager over the lower abdomen of the patient based on the sensor data.

7. The imaging system of any one of claims 1 to 6, wherein the AI algorithm comprises a real-time anatomical feature detection model that is configured to identify the placenta, cervix and the maternal urinary bladder.

8. The imaging system of claim 7, wherein the anatomical feature detection model comprises a single shot detection algorithm.

9. The imaging system of any one of claims 1 to 8, wherein the AI algorithm comprises a segmentation model that is configured to segment the placenta, cervix and uterus.

10. The imaging system of claim 9, wherein the AI algorithm is configured to identify a position of the internal OS as a point on the periphery of the cervix segmentation.

11. The imaging system of claim 10, wherein the segmentation model is configured to identify the internal OS and determine the placenta to internal OS distance by:
defining a bounding box around the cervix segmentation;
forming a diagonal line through the bounding box;
identifying where the diagonal line meets the cervix segmentation boundary in a central imaging slice of the cervix, and defining this point as the internal OS;
defining a sphere around the internal OS; and
determining if the placenta is within the sphere using the placenta segmentation.

12. A placental imaging method comprising:
receiving image data from a hand held ultrasound imager during scanning over the lower abdomen of a patient;
using an AI algorithm to:
perform segmentation to identify the location and shape of the placenta and cervix within the image data;
use the cervix location and shape to identify a position of the internal OS;
use the placenta location and shape to determine the placenta to internal OS distance; and
generate a placental positional abnormality indication;
receiving a gestational age of the patient and the placental positional abnormality indication; and
recommending a patient action based on the gestational age and the placental positional abnormality indication.

13. The method of claim 12, further comprising receiving sensor data that indicates the orientation and speed of movement of the hand held ultrasound imager and the method comprises performing an image quality check by comparing the sensor data with a scan protocol.

14. The method of claim 12 or 13, wherein using the AI algorithm comprises:
using a real-time anatomical feature detection model to identify the placenta and cervix;
using a segmentation model to segment the placenta, cervix and uterus and identify the internal OS.

15. A computer program comprising computer program code which is adapted, when said program is run on a computer, to implement the method of any one of claims 12 to 14.
